(19) Europäisches Patentamt
European Patent Office
Office européen des brevets

(11) Numéro de publication: 0 229 566 B1

(12) # FASCICULE DE BREVET EUROPEEN

(45) Date de publication de fascicule du brevet: **06.05.92**

(51) Int. Cl.[5]: **C07D 207/48**, A61K 31/40, C07D 401/12, C07D 405/12, C07D 409/12

(21) Numéro de dépôt: **86402906.1**

(22) Date de dépôt: **23.12.86**

Le dossier contient des informations techniques présentées postérieurement au dépôt de la demande et ne figurant pas dans le présent fascicule.

(54) **Dérivés de la 1-benzène sulfonyl 2-oxo 5-alcoxy pyrrolidine, leur procédé de préparation, leur application comme médicaments et les compositions les renfermant.**

(30) Priorité: **30.12.85 IT 2340685**
**11.07.86 IT 2110586**

(43) Date de publication de la demande:
**22.07.87 Bulletin 87/30**

(45) Mention de la délivrance du brevet:
**06.05.92 Bulletin 92/19**

(84) Etats contractants désignés:
**AT BE CH DE ES FR GB GR IT LI LU NL SE**

(56) Documents cités:
**EP-A- 0 138 721**
**US-A- 3 453 289**
**US-A- 4 217 130**

(73) Titulaire: **ROUSSEL-UCLAF**
**35, Boulevard des Invalides**
**F-75007 Paris(FR)**

(72) Inventeur: **Toja, Emilio**
**Via Plezzo 80**
**I-20100 Milano(IT)**
Inventeur: **Gorini, Carlo**
**Via Orcagna 4**
**I-20100 Milano(IT)**
Inventeur: **Zirotti, Carlo**
**Via 2 Giugno 12**
**I-Arona (NO)(IT)**
Inventeur: **Barzaghi, Fernando**
**Via Monteverdi 21**
**I-20052 Monza(IT)**
Inventeur: **Galliani, Giulio**
**Via Silva 13**
**I-20052 Monza (MI)(IT)**

(74) Mandataire: **Tonnellier, Marie-José et al**
**Département des Brevets ROUSSEL UCLAF**
**B.P. no 9**
**F-93230 Romainville(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 229 566 B1

## Description

L'invention concerne de nouveaux dérivés de la l-(benzènesulfonyl) 2-oxo 5-alcoxy pyrrolidine, leur procédé de préparation, leur application comme médicaments et les compositions les renfermant.

La demande de brevet européen EP-A 138721(A) décrit des 4-hydroxy ou 4-acyloxy 1-(benzènesulfonyl) 2-oxo pyrrolidines qui peuvent être utilisés comme médicaments notamment dans le traitement des troubles de la mémoire et de l'amnésie.

L'invention a pour objet les composés répondant à la formule générale (I) :

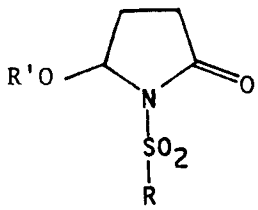

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical furyle, pyrannyle, pyridyle, benzofurannyle, isobenzofurannyle, chromanyle, isochromanyle, chroményle, xanthényle, phénoxathiinyle, oxazolyle, isoxazolyle, furazannyle, phénoxazinyle, thiéno[2,3-b] furannyle, 2H-furo[3,2-b]pyrannyle, benzoxazolyle ou morpholinyle, R étant éventuellement substitué par un ou plusieurs substituants choisis dans le groupe constitué par le radical hydroxyle, le radical acétoxy, le radical méthoxy ou le radical benzyloxy, le radical méthyle, éthyle, propyle ou isopropyle, le radical éthényle ou le radical éthynyle, le fluor, le chlore, le brome, les groupements $CF_3$, $SCF_3$, $OCF_3$, $NO_2$, $NH_2$ ou $C{\equiv}N$, le radical phényle, les groupements acyles et alcoxycarbonyles renfermant de 2 à 8 atomes de carbone et les groupements alcoylsulfonyles renfermant de 1 à 6 atomes de carbone.

Par radical alcoyle, on entend de préférence un radical renfermant de 1 à 5 atomes de carbone, par exemple, le radical méthyle, éthyle, propyle, isopropyle, butyle, isobutyle, tertbutyle, pentyle, hexyle, cyclopropyle,cyclobutyle, cyclopentyle ou cyclohexyle.

Par radical alcényle, on entend de préférence un radical éthényle, propényle, butényle.

Par radical acyle, on entend de préférence un radical acétyle, propionyle ou butyryle.

L'invention a notamment pour objet les composés de formule (I) dans lesquels R représente un radical phényle éventuellement substitué par un radical méthoxy.

L'invention a aussi notamment pour objet les composés de formule (I) dans lesquels R' représente un radical alcoyle linéaire, ramifié ou cyclique, renfermant jusqu'à 8 atomes de carbone et plus particulièrement un radical éthyle.

L'invention a tout spécialement pour objet la l-(benzènesulfonyl) 2-oxo 5-éthoxy pyrrolidine.

Parmi les composés de l'invention, on peut encore citer la 1-(4-nitro benzènesulfonyl) 2-oxo 5-éthoxy pyrrolidine, la 1-(benzènesulfonyl) 2-oxo 5-isopropoxy pyrrolidine, la 1-(benzènesulfonyl) 2-oxo 5-propoxy pyrrolidine.

Les composés de l'invention présentent d'intéressantes propriétés pharmacologiques : ils retardent l'extinction de la réponse d'évitement conditionné, ils retardent la disparition de la réponse apprise. Ils favorisent l'attention, la vigilance et la mémorisation.

L'invention a donc pour objet les produits de formule (I) en tant que médicaments, utiles notamment dans le traitement des asthénies intellectuelles ou nerveuses, des défaillances de la mémoire, de la sénescence, du surmenage intellectuel.

L'invention a plus particulièrement pour objet en tant que médicament, le produit de l'exemple 1, ainsi que les produits des exemples 14, 35 et 36.

La posologie usuelle est variable selon l'affection en cause, le sujet traité et la voie d'administration, elle peut être comprise entre 50 mg et 3000 mg/jour, par exemple entre 150 et 1500 mg/jour en une ou plusieurs prises pour le produit de l'exemple 1 administré par voie orale.

La présente invention a également pour objet les compositions pharmaceutiques renfermant comme principe actif au moins un produit de formule (I). Les compositions pharmaceutique de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) :

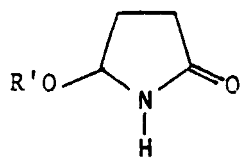

(II)

dans laquelle R' conserve la même signification que précédemment, à l'action d'un composé de formule (III) :

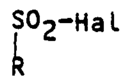

(III)

dans laquelle Hal représente un atome de chlore ou de brome et R conserve la même signification que précédemment, pour obtenir le composé de formule (I) correspondant.

Dans un mode de réalisation préférée du procédé de l'invention, la réaction entre le produit de formule (II) et le produit de formule (III) est effectuée :

a) en présence d'une base forte comme le butyllithium, un hydrure alcalin, comme l'hydrure de sodium ou le bis-(triméthylsilyl) amidure de sodium ;

b) au sein d'un solvant choisi dans le groupe constitué par le tétrahydrofuranne, le benzène, le diméthylformamide, le diméthylsulfoxyde, l'éther monoéthylique du diéthylène glycol, ou l'éther diéthylique du diéthylène glycol.

L'invention a également pour objet un procédé tel que défini précédemment, pour la préparation des composés de formule (I) dans laquelle R représente un radical phényle ou naphtyle substitué par un ou plusieurs radicaux amino ou hydroxy, caractérisé en ce que l'on réduit les composés de formule (I) correspondants dans laquelle R représente respectivement un ou plusieurs radicaux nitro, méthoxy ou benzyloxy.

Dans un mode de réalisation préférée du procédé ci-dessus, la réduction est effectuée par l'hydrogène en présence d'un catalyseur à base de palladium, au sein d'un solvant organique qui est de préférence l'éthanol.

L'invention a également pour objet un procédé de préparation des composés de formule (I), dans laquelle R est défini comme précédemment et R' représente un atome d'hydrogène ou un radical acyle renfermant de 1 à 6 atomes de carbone, caractérisé en ce que l'on soumet un halogénure de 4-penténoyle de formule (IV) :

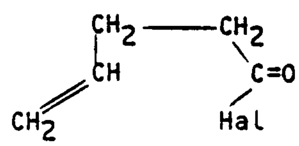

(IV)

dans laquelle Hal représente un atome d'halogène, en présence d'un agent de condensation, à l'action d'un composé de formule (V) :

$H_2N$-$SO_2$-R (V)

pour obtenir un composé de formule (VI) :

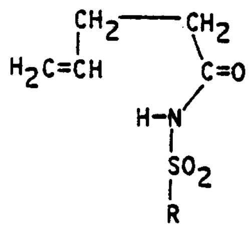

(VI)

que l'on cyclise pour obtenir un composé de formule (I) correspondant, dans laquelle R′ représente un atome d'hydrogène :

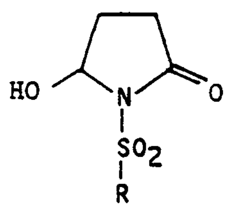

(I avec R' = H)

que l'on acyle si désiré pour obtenir un composé de formule (I) correspondant, dans laquelle R′ représente un radical acyle :

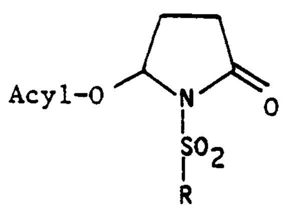

(I avec R' = Acyle)

Dans un mode de réalisation préféré du procédé ci-dessus :

- la réaction du composé de formule (IV) avec le composé de formule (V) est effectuée en présence d'oxychlorure de phosphore ou de chlorure de thionyle ;
- la cyclisation du composé de formule (VI) est effectuée en présence de tétraoxyde d'osmium, puis de métapériodate de sodium ou par action de l'ozone ;
- les éventuels substituants réactifs du radical R sont protégés lors de la condensation et de la cyclisation puis déprotégés ensuite, selon des méthodes connues de l'homme de l'art.

Certaines préparations des produits de départ des procédés de l'invention sont fournis ci-après dans la partie expérimentale.

Les produits de formule (II) utilisés comme produits de départ sont des produits connus qui peuvent être préparés selon le procédé décrit dans Tétrahedron 31, 1437 (1975) ou Tétrahedron 41, 2007 (1985).

Les exemples suivants illustrent l'invention sans toutefois la limiter.

**Exemple 1 : 1-benzènesulfonyl 2-oxo 5-éthoxy pyrrolidine.**

A 4,5 g de 5-éthoxy pyrrolidine 2-one dissous dans 140 cm3 de tétrahydrofuranne anhydre, on ajoute goutte à goutte sous atmosphère inerte, 21,8 cm3 de butyllithium dans l'hexane (1,6 M) en refroidissant à -10¤C environ. Après 45 minutes, on ajoute 6,15 g de chlorure de benzènesulfonyle dans du tétrahydrofuranne, maintient à -10¤C sous agitation pendant 2 heures puis laisse revenir à température ambiante. On

concentre sous pression réduite, reprend avec de l'éthanol 100. Par refroidissement et sous agitation, le produit précipite. On filtre, lave abondamment à l'eau, sèche et obtient 2,8 g de produit attendu après cristallisation dans l'isopropanol. F = 112-113¤C.

| Analyse : | | | |
|---|---|---|---|
| Calculé :<br>Trouvé: | C% 53,52<br>53,30 | H% 5,61<br>5,64 | N% 5,20<br>5,10 |

**Exemple 2 : 1-(4-méthoxybenzènesulfonyl) 2-oxo 5-éthoxy pyrrolidine.**

On opère comme à l'exemple 1 à partir de 5,2 g de chlorure de paraméthoxy benzènesulfonyle et obtient le produit attendu que l'on chromatographie sur silice (éluant : benzène-acétate d'éthyle 5-2). On obtient 1,5 g de produit attendu. F = 112-113¤C après recristallisation dans l'isopropanol.

| Analyse : | | | |
|---|---|---|---|
| Calculé:<br>Trouvé: | C% 52,16<br>52,21 | H% 5,72<br>5,71 | N% 4,68<br>4,62 |

**Exemples 3 à 46 :**

En opérant de manière analogue à celle décrite à l'exemple 1 ou à l'exemple 2, on a préparé les produits figurant dans les tableaux ci-après. Dans ces tableaux sont indiquées les constantes physiques et analyses des produits, ainsi que les principales caractéristiques des modes opératoires utilisés.

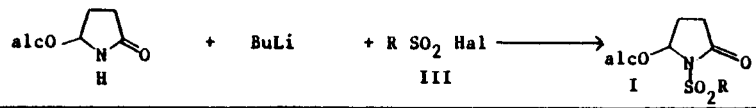


| Ex. | alc | R | + BuLi | + III | Obtention de I | | solv. crist. | rendt. | F°C | Analyse | | | |
|---|---|---|---|---|---|---|---|---|---|---|---|---|---|
| | | | | | | Chromato | | | | | C % | H % | N % |
| 3 | Et | 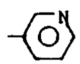 | - 25°C<br>25 mn | - 38°C<br>20 mn | - | toluène 1<br>$CH_3CO_2Et$ 1 | isopropanol | 46% | 59-61 | Calculé<br>Trouvé | 48,87<br>48,69 | 5,22<br>5,08 | 10,36<br>10,17 |
| 4 | " | 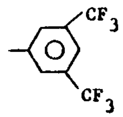 | - 40°C<br>25 mn | - 40°C<br>↗ t.a. | - | toluène 8<br>$CH_3CO_2Et$ 2 | " | 34% | 77-79 | | 41,48<br>41,54 | 3,23<br>3,14 | 3,45<br>3,43 |
| 5 | " | 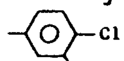 | - 30°C<br>30 mn | - 30°C<br>↗ t.a. | +EtOH↓ | - | " | 42% | 110-111 | | 42<br>42,37 | 3,52<br>3,43 | 3,76<br>3,69 |
| 6 | " | 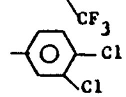 | - 20°C<br>35 mn | - 20°C<br>↗ t.a. | - | toluène 8<br>$CH_3CO_2Et$ 2 | " | 23% | 104-105 | | 42,61<br>42,47 | 3,87<br>3,77 | 4,14<br>4,05 |
| 7 | " | 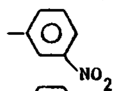 | - 20°C<br>40 mn | - 20°C<br>↗ t.a. | | " | " | 22,8% | 83-84 | | 45,85<br>46,09 | 4,48<br>4,61 | 8,91<br>8,98 |
| 8 | " | 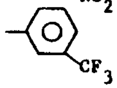 | - 15°C<br>40 mn | - 40°C<br>↗ t.a. | - | toluène 2<br>$CH_3CO_2Et$ 8 | cyclohexane | 23% | 58-59 | | 46,29<br>46,08 | 4,18<br>4,07 | 4,15<br>4,37 |
| 9 | " | 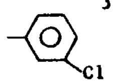 | - 15°C<br>40 mn | - 28°C<br>1 h | - | benzène 8<br>$CH_3CO_2Et$ 2 | isopropanol | 28% | 75-77 | | 47,44<br>47,39 | 4,64<br>4,69 | 4,61<br>4,55 |
| 10 | " | 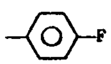 | - 15°C<br>40 mn | - 20°C<br>↗ t.a. | - | toluène 8<br>$CH_3CO_2Et$ 2 | " | 40% | 113-115 | | 50,16<br>50,25 | 4,91<br>4,99 | 4,87<br>4,86 |
| 11 | " | 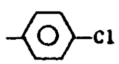 | - 25°C<br>40 mn | - 25°C<br>↗ t.a. | +EtOH↓ | - | " | 27% | 120-122 | | 47,44<br>47,52 | 4,64<br>4,66 | 4,61<br>4,56 |
| 12 | " | 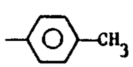 | - 15°C<br>40 mn | - 20°C<br>↗ t.a. | - | toluène 8<br>$CH_2CO_2Et$ 2 | " | 28,6% | 147-149 | | 55,10<br>55,28 | 6,05<br>6,21 | 4,94<br>5,12 |

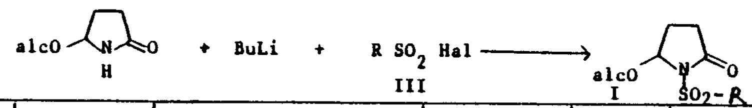


| Ex. | alc | R | + BuLi | + III | Obtention de I | | solv. crist. | Rendt | F°C | Analyse | | | |
|---|---|---|---|---|---|---|---|---|---|---|---|---|---|
| | | | | | | Chromato | | | | | C % | H % | N % |
| 13 | Et | 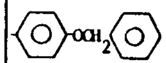 | - 20°C<br>20 mn | - 20°C<br>↗ t.a. | + $H_2O$ ↓ | toluène 8<br>$CH_3CO_2Et$ 2 | isopropanol | 28,6% | 147-149 | Calculé<br>Trouvé | 55,10<br>55,28 | 6,05<br>6,21 | 4,94<br>5,12 |
| 14 | " | 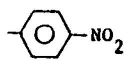 | - 20°C<br>35 mn | - 50°C<br>30 mn | + $H_2O$ ↓ | - | EtOH | 51% | 125-126 | | 45,85<br>45,93 | 4,48<br>4,44 | 8,91<br>9,06 |
| 15 | " | 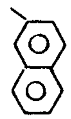 | - 20°C<br>20 mn | - 20°C<br>1 h | + $H_2O$ ↓ | - | " | 28% | 110 | | 60,17<br>60,07 | 5,36<br>5,22 | 4,38<br>4,46 |
| 16 | " | 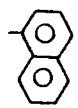 | - 20°C<br>20 mn | - 20°C<br>↗ t.a. | + $H_2O$ ↓ | - | isopropanol | 39% | 139-140 | | 60,17<br>60,12 | 5,36<br>5,31 | 4,38<br>4,22 |
| 17 | " | 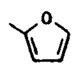 | - 30°C<br>20 mn | - 40°C<br>↗ t.a. | +MeOH | toluène 8<br>$CH_3CO_2Et$ 2 | isopropanol | 29% | 74-76 | | 46,34<br>46,49 | 5,05<br>5,04 | 5,40<br>5,42 |
| 18 | " | 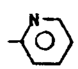 | - 20°C<br>20 mn | - 20°C<br>15 mn | - | " | $CHCl_3$ | 10% | 59-60 | | 48,61<br>48,69 | 5,16<br>5,08 | 10,29<br>10,17 |
| 19 | " | 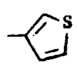 | - 25°C<br>20 mn | - 36°C<br>↗ t.a. | - | " | isopropanol | 35% | 98-99 | | 43,62<br>43,29 | 4,75<br>4,68 | 5,08<br>5,16 |
| 20 | " | 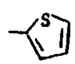 | - 20°C<br>35 mn | - 30°C<br>2 h 30 | - | benzène 8<br>$CH_3CO_2Et$ 2 | " | 28% | 100-102 | | 43,57<br>43,62 | 4,82<br>4,75 | 5,02<br>5,08 |

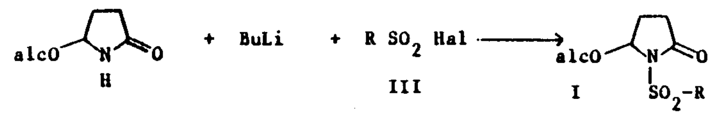


| Ex. | Alc | R | + BuLi | + III | Obtention de I | | solv. crist. | Rendt | F°C | Analyse | | | |
|---|---|---|---|---|---|---|---|---|---|---|---|---|---|
| | | | | | | Chromato | | | | | C % | H % | N % |
| 21 | Et | 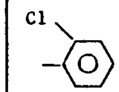 | - 20°C<br>15 mn | - 20°C<br>1 h | + $H_2O$↓ | - | isopropanol | 18,6% | 100-101 | Calculé<br>Trouvé | 47,63<br>47,44 | 4,72<br>4,64 | 4,53<br>4,61 |
| 22 | " | 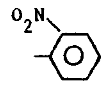 | - 20°C<br>20 mn | - 20°C<br>↗ t.a. | - | toluène 8<br>$CH_3CO_2Et$ 2 | " | 22,6% | 98-100 | | 45,85<br>46,01 | 4,48<br>4,39 | 8,91<br>8,83 |
| 23 | " | 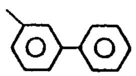 | - 20°C<br>15 mn | - 35°C<br>1 h | + $H_2O$ \|<br>→ $CHCl_3$ | " | " | 36% | 89-90 | | 62,48<br>62,58 | 5,69<br>5,54 | 4,31<br>4,05 |
| 24 | " | 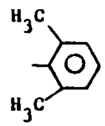 | - 23°C<br>20 mn | - 27°C<br>↗ t.a. | EtOH ↓ | - | " | 25,5% | 106-107 | | 56,54<br>56,32 | 6,43<br>6,39 | 4,71<br>4,59 |
| 25 | " | 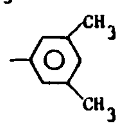 | - 25°C<br>20 mn | - 37°C<br>↗ t.a. | EtOH ↓ | - | " | 35% | 100-102 | | 56,54<br>56,31 | 6,43<br>6,50 | 4,71<br>4,65 |
| 26 | " | 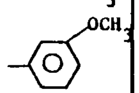 | - 35°C<br>20 mn | - 35°C<br>↗ t.a. | - | toluène 8<br>$CH_3CO_2Et$ 2 | - | 43% | - | | 52,16<br>51,88 | 5,72<br>5,66 | 4,67<br>4,56 |
| 27 | " | 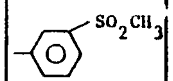 | - 20°C<br>35 mn | - 25°C<br>1 h | + $H_2O$ | - | EtOH | 17,7% | 153-154 | | 45,12<br>44,94 | 4,96<br>4,98 | 4,22<br>4,03 |
| 28 | " | 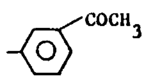 | - 20°C<br>15 mn | - 20°C<br>1 h | + $H_2O$↓<br>→ $CHCl_3$ | toluène 6<br>$CH_3CO_2Et$ 4 | isopropanol | - | - | | 54,00<br>54,12 | 5,50<br>5,48 | 4,50<br>4,37 |

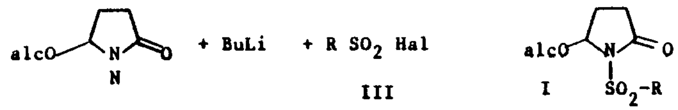


| Ex. | alc | R | + BuLi | + III | Obtention de I | | solv. crist. | Rendt | F°C | Analyse | | | |
|---|---|---|---|---|---|---|---|---|---|---|---|---|---|
| | | | | | | Chromato | | | | | C % | H % | N % |
| 29 | Et | 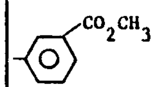 | - 28°C<br>20 mn | - 42°C<br>↗ t.a. | EtOH↓ | - | isopropanol | 29% | 101-102 | Calculé<br>Trouvé | 51,36<br>51,43 | 5,23<br>5,17 | 4,27<br>4,16 |
| 30 | " | 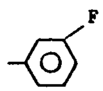 | - 30°C<br>20 mn | - 38°C<br>↗ t.a. | - | toluène 8<br>$CH_3CO_2Et$ 2 | " | 33,6% | 57-59 | | 50,16<br>49,94 | 4,91<br>4,93 | 4,87<br>4,73 |
| 31 | " | 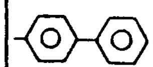 | - 25°C<br>20 mn | - 35°C<br>↗ t.a. | - | toluène 8<br>$CH_3CO_2Et$ 2 | " | 36% | 117-120 | | 62,58<br>62,77 | 5,54<br>5,59 | 4,05<br>3,97 |
| 32 | " | 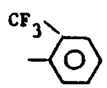 | - 35°C<br>20 mn | - 35°C<br>↗ t.a. | - | " | " | 29% | 93-94 | | 46,29<br>46,41 | 4,18<br>4,21 | 4,15<br>4,19 |
| 33 | " | 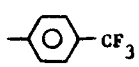 | - 30°C<br>20 mn | - 35°C<br>↗ t.a. | EtOH$H_2O$↓ | - | " | 26% | 99-101 | | 46,29<br>46;04 | 4,18<br>4,32 | 4,15<br>4,06 |
| 34 | $CH_3$ | 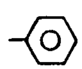 | - 10°C<br>30 mn | - 27°C<br>↗ t.a. | +$CH_3OH$↓ | - | " | 28% | 121-123 | | 51,75<br>52,03 | 5,13<br>5,24 | 5,49<br>5,58 |
| 35 | $-CH(CH_3)_2$ | 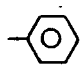 | " | " | + $H_2O$↓ | - | EtOH | 61% | 158-160 | | 55,10<br>55,26 | 6,05<br>6,17 | 4,94<br>5,01 |
| 36 | $-(CH_2)_2CH_3$ | 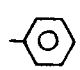 | - 18°C<br>30 mn | - 30°C<br>↗ t.a. | - | $CH_3CO_2Et$ 1<br>n-hexane 1 | cyclohecane 1<br>éther iso 1 | 61% | 63,65 | | 55,10<br>55,29 | 6,05<br>6,03 | 4,91<br>4,79 |

alcO ... H

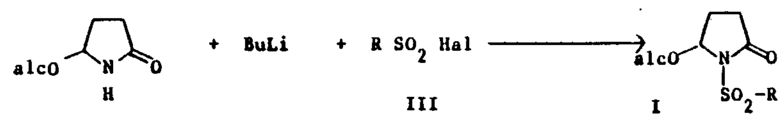


| Ex. | alc | R | + BuLi | + III | Obtention de I | | solv. crist. | Rendt | F°C | Analyse | | | |
|---|---|---|---|---|---|---|---|---|---|---|---|---|---|
| | | | | | | Chromato | | | | | C% | H% | N% |
| 37 | $-CH(CH_3)_2$ | 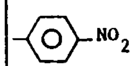 | - 35°C 20 mn | - 35°C ↗ t.a. | + $H_2O$ ↓ | $CH_3CO_2Et$ 1 n-hexane 2 | EtOH | 42,9% | 153-154 | Calculé Trouvé | 47,55 47,39 | 4,91 4,88 | 8,53 8,61 |
| 38 | " | 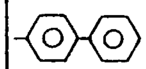 | - 35°C 30 mn | - 35°C 30 mn | + $H_2O$ ↓ | - | EtOH | 43,02% | 135-137 | | 63,49 63,41 | 5,89 5,83 | 3,90 3,97 |
| 39 | $-(CH_2)_3CH_3$ | 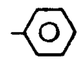 | - 47°C 30 mn | - 47°C ↗ t.a. | - | $CH_3CO_2Et$ | éther iso. n-hexane | 31,70% | 46-48 | | 56,54 56,61 | 6,44 6,39 | 4,71 4,68 |
| 40 | 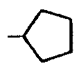 | 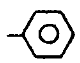 | - 32°C 20 mn | - 32°C ↗ t.a. | + $H_2O$ ↓ | - | 1) isopropanol 2) EtOH | 54,7% | 96-97 | | 58,23 58,05 | 6,19 6,16 | 4,53 4,76 |
| 41 | 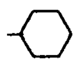 | 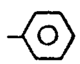 | " | " | " | - | " | 28% | 108-109 | | 59,42 59,21 | 6,54 6,66 | 4,33 4,29 |
| 42 | $-(CH_2)_4CH_3$ | 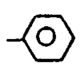 | - 35°C | - 40°C ↗ t.a. | - | $CH_3CO_2Et$ 1 n-hexane 2 | - | 75,7% | - | | 57,85 58,02 | 6,80 6,74 | 4,50 4,63 |
| 43 | $-(CH_2)_5CH_3$ | 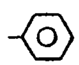 | " | " | - | " | - | 32% | eb 250°C 0,08mbar | | 59,05 58,94 | 7,12 6,99 | 4,30 4,15 |
| 44 | $-CH_2CH(CH_3)_2$ | 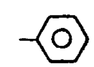 | " 20 mn | - 45°C ↗ t.a. | | " | - | 67,2% | 70-71 | | 56,54 56,36 | 6,44 6,51 | 4,71 4,76 |
| 45 | $-CH(CH_3)_2$ | 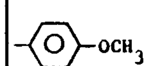 | " | " | + $H_2O$ ↓ | - | isopropanol | 52,9% | 97-98 | | | | |
| 46 | " | 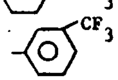 | " | " | " | - | - | 24,4% | 101-102 | | | | |
| 46 | " | 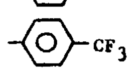 | " | " | " | - | - | - | - | | | | |

**Exemple 47 : 1-(4-hydroxybenzènesulfonyl) 2-oxo 5-éthoxy pyrrolidine.**

On hydrogène, à 20¤C sous 1000 mbar, 6,3 g de 1-(4-benzyloxyphénylsulfonyl) 5-éthoxy pyrrolidin-2-one en présence de 1,2 g de catalyseur (Pd à 10%), dans 150 cm3 d'éthanol à 96¤. On filtre, concentre le filtrat et cristallise le résidu dans l'éthanol 96¤. On obtient 2,5 g de produit attendu. F = 162-163¤C.

| Analyse : | | | |
|---|---|---|---|
| Calculé:<br>Trouvé: | C% 50,51<br>50,70 | H% 5,30<br>5,21 | N% 4,90<br>5,01 |

**Exemple 48 : 1-(4-aminobenzènesulfonlyl) 2-oxo 5-éthoxy pyrrolidine.**

On hydrogène à température et pression ambiantes 4,5 g de 1-(4-nitrophénylsulfonyl) 5-éthoxy pyrrolidin-2-one dissous dans 200 cm3 d'éthanol 96¤ en présence de palladium à 10% sur carbone. On filtre, reprend l'insoluble avec du chloroforme chaud, filtre et ajoute le dernier filtrat à la solution alcoolique issue de la première filtration. On concentre à sec sous pression réduite. On cristallise le résidu 2 fois dans l'éthanol 96¤ et obtient 2,3 g de produit attendu. F = 185-186¤C.

| Analyse : | | | |
|---|---|---|---|
| Calculé:<br>Trouvé: | C% 50,69<br>50,45 | H% 5,67<br>5,57 | N% 9,85<br>9,79 |

**Exemple 49 : 1-benzènesulfonyl 2-oxo 5-benzyloxy pyrrolidine.**

A une solution de 4,64 g de sodium bis-(triméthylsilyl) amide dans 350 cm3 d'éther éthylique anhydre, on ajoute 4,40 g de 5-benzyloxy pyrrolidin-2-one à température ambiante et sous atmosphère inerte. On agite 30 minutes, refroidit à 0¤C et ajoute une solution de 4,06 g de chlorure de benzènesulfonyle dans 30 cm3 d'éther anhydre en maintenant la température à 0¤C. On laisse revenir à température ambiante, on filtre et évapore le solvant sous pression réduite. On chromatographie le résidu sur silice, élue par un mélange acétate d'éthyle-n-hexane (1-2). Après recristallisation dans un mélange benzène-n-hexane (1-3), on obtient 2,25 g de produit attendu. F = 80-81¤C.

| Analyse : $C_{17}H_{17}NO_4S$ | | | |
|---|---|---|---|
| Calculé:<br>Trouvé: | C% 61,61<br>61,39 | H% 5,17<br>5,23 | N% 4,23<br>4,31 |

**Exemple 50 : 1-benzènesulfonlyl 2-oxo 5-(propèn-2-yloxy) pyrrolidine.**

On opère comme à l'exemple 49 à partir de 0,80 g de 5-(2-propènyloxy) pyrrolidin-2-one. On refroidit à -10¤C et on ajoute une solution de 1,00 g de chlorure de benzènesulfonyle dans 10 cm3 d'éther anhydre en maintenant la température à -10C. On ajoute 100 cm3 d'acétate d'éthyle avant de filtrer. On élue par un mélange acétate d'éthyle-hexane (1-1) et cristallise dans l'éther isopropylique pour obtenir 0,20 g. F = 54-56¤C.

| Analyse : $C_{13}H_{15}NO_4S$ | | | |
|---|---|---|---|
| Calculé:<br>Trouvé: | C% 55,50<br>55,62 | H% 5,37<br>5,28 | N% 4,98<br>5,01 |

**Préparation de la 5-(2-propényloxy) pyrrolidin-2-one utilisée au départ de l'exemple 50.**

On chauffe à 65¤C pendant 1 heure 30 minutes 2,5 g de 5-hydroxy pyrrolidin-2-one et 1,25 g de résine Amberlite IR 120 (H) dans 55 cm3 d'alcool allylique. On laisse refroidir à température ambiante, évapore l'alcool sous pression réduite et chromatographie le résidu sur silice en éluant à l'acétate d'éthyle. On obtient 0,90 g de produit attendu.

| Analyse : $C_7H_{11}NO_2$ | | | |
|---|---|---|---|
| Calculé:<br>Trouvé: | C% 59,56<br>57,81 | H% 7,85<br>7,73 | N% 9,92<br>9,10 |

**Exemple 51 : 1-benzènesulfonyl 2-oxo 5-hydroxypyrrolidine.**

A une solution de 5 g de N-benzènesulfonyl 4-penténoylamide dans 150 cm3 de dioxanne et 50 cm3 d'eau, on ajoute 50 mg de tétraoxyde d'osmium. Après environ 20 minutes, on ajoute 5,10 g de métapériodate de sodium. On agite 1 heure et ajoute à nouveau 5,10 g de métapériodate de sodium puis agite encore 2 heures à température ambiante. On filtre le précipité formé, évapore à sec le filtrat et dissout le résidu dans 50 cm3 d'acétate d'éthyle. On traite au charbon actif puis évapore à sec. On triture le résidu dans l'éther éthylique anhydre et obtient 2,05 g de produit attendu que l'on cristallise dans l'isopropanol. F = 121¤C.

| Analyse : $C_{10}H_{11}NO_4S$ PM : 241,27 | | | |
|---|---|---|---|
| Calculé:<br>Trouvé: | C% 49,78<br>49,69 | H% 4,59<br>4,46 | N% 5,80<br>5,89 |

Le N-benzènesulfonyl 4-penténoylamide utilisé au départ de l'exemple 51 a été préparé comme suit.

On agite 20 heures à température ambiante 2,4 g de chlorure de 4-penténoyle (Tétrahedron 32, 1085 (1976) et 2,65 g de benzènesulfonamide dans 5 cm3 d'oxychlorure de phosphore. On évapore à sec soigneusement la solution obtenue sous pression réduite et obtient 4 g de produit attendu. Ce produit est utilisé tel quel.

**Exemple 52 : 1-benzènesulfonyl 2-oxo 5-acétoxypyrrolidine.**

On chauffe au reflux pendant 1 heure et demie 2,90 g de 1-benzène sulfonyl 5-hydroxy pyrrolidin-2-one dans 58 cm3 d'anhydride acétique. On laisse refroidir à température ambiante et évapore le solvant sous pression réduite en formant l'azéotrope anhydride acétique-toluène. On cristallise le résidu dans l'éthanol 95¤ et obtient 2,45 g de produit attendu. F = 153-155¤C.

| Analyse : $C_{12}H_{13}NO_5S$ PM : 283,31 | | | |
|---|---|---|---|
| Calculé:<br>Trouvé: | C% 50,85<br>51,04 | H% 4,62<br>4,49 | N% 4,94<br>5,12 |

**Préparation de la 5-isopropoxy pyrrolidin-2-one utilisée au départ des exemples 35 et 37.**

On refroidit à -10¤C, 28,64 g de succinimide dans 1200 cm3 d'isopropanol, ajoute 32,80 g de borohydrure de sodium, agite 4 heures en maintenant la température entre -10¤C et 0¤C et en ajoutant toutes les 15 minutes 15 gouttes d'une solution 2 N d'acide chlorhydrique dans l'isopropanol. Puis entre 0 et 2¤C, on ajuste le pH à 2-3 en ajoutant une solution 2 N d'acide chlorhydrique dans l'isopropanol et agite 2 heures à cette température. Enfin, on neutralise à 0¤C à l'aide d'une solution d'hydroxyde de potassium dans l'isopropanol. On évapore le solvant sous pression réduite, extrait au chloroforme, concentre à sec sous pression réduite et obtient 20,5 g de produit attendu. F = 68-71¤C.

**Préparation de la 5-propoxy pyrrolidin-2-one utilisée au départ de l'exemple 36.**

On opère d'une manière analogue avec le n-propanol au lieu de l'isopropanol. On emploie 16 g de borohydrure de sodium et refroidit à -7¤-0¤C. On obtient 27,50g de produit attendu. F = 52-54¤C.

**Préparation de la 5-méthoxy pyrrolidin-2-one utilisée au départ de l'exemple 34 et de la 5-butoxy pyrrolidin-2-one utilisée au départ de l'exemple 39.** Synthesis (4) 315-317 (1980) (technique au borohydrure de sodium).

**Préparation du chlorure de benzyloxyphénylsulfonyle utilisée au départ de l'exemple 13.**

**a) Benzyloxyphénylsulfonate de sodium.**

On agite pendant 30 minutes à température ambiante 30 g de sel de sodium de l'acide paraphénol sulfonique bihydraté dans 48 cm3 de solution aqueuse d'hydroxyde de sodium à 15%. On ajoute une solution de 27,95 g de bromure de benzyle dans 60 cm3 d'éthanol et chauffe au reflux pendant 5 heures. On laisse reposer une nuit puis filtre 34,3 g de produit attendu.

**b) Chlorure de benzyloxyphénylsulfonyle.**

On mélange 34,3 g de produit obtenu ci-dessus, 430 cm3 de chlorure de méthylène et ajoute 24,95 g de pentachlorure de phosphore et chauffe au reflux pendant 7 heures. On amène à sec sous pression réduite et extrait au toluène anhydre. On évapore le solvant et recristallise le résidu dans l'éther isopropylique et filtre à chaud sur charbon actif. On obtient 23,95 g de produit attendu. F = 101-103¤C

**Préparation du 5-isobutoxy pyrrolidin-2-one.**

On chauffe à 65¤C pendant 3 heures 2,5 g de 5-hydroxy pyrrolidin-2-one et 1,25 g de résine Amberlite IR-120 (H) dans 55 cm3 d'isobutanol. On laisse revenir à température ambiante, filtre la résine et distille le solvant sous pression réduite. On chromatographie le résidu sur silice, élue à l'acétate d'éthyle et obtient 2,90 g de produit attendu. F = 30-32¤C.

| Analyse : $C_8H_{15}NO_2$ | | | |
|---|---|---|---|
| Calculé: | C% 61,12 | H% 9,62 | N% 8,91 |
| Trouvé: | 60,87 | 9,54 | 8,83 |

Le 5-pentyloxy pyrrolidin-2-one, le 5-cyclopentyloxy pyrrolidin-2-one, le 5-benzyloxy pyrrolidin-2-one, le 5-hexyloxy pyrrolidin-2-one, le 5-cyclohexyloxy pyrrolidin-2-one ; ces produits ont aussi été préparés comme ci-dessus en utilisant la résine Amberlite et l'alcool approprié. (Voir tableau ci-après).

| R | réaction | crist. | chromato | F°C | crist. | Rendt. |
|---|---|---|---|---|---|---|
| pentyle | 65°C 3 h | | $CH_3CO_2Et$ | 42-43 | | 63,4% |
| cyclopentyle | " " | | " | 87-89 | 90-91°C isopropanol | 61,7% |
| benzyle | 60°C 3 h | n-hexane 3 benz 1 | - | 80-82 | | 72,1% |
| hexyle | " " | | $CH_3CO_2Et$ | | | 68,2% |
| cyclohexyle | " " | | $CH_3CO_2Et$ | 85-7 | 95-96°C | 41,38% |

## Exemples de compositions pharmaceutiques.

a) On a préparé des comprimés répondant à la formule suivante :
- Produit de l'exemple 1 ......................... 100 mg
- Excipient q.s. pour un comprimé terminé à ...... 300 mg.

(Détail de l'excipient : lactose, amidon de blé, amidon traité, amidon de riz, stéarate de magnésium, talc).

b) On a préparé des gélules répondant à la formule suivante :
- Produit de l'exemple 1 ......................... 200 mg
- Excipient q.s. pour une gélule terminée à ...... 300 mg.

(Détail de l'excipient : talc, stéarate de magnésium, aérosil).

## ETUDE PHARMACOLOGIOUE

### Toxicité aiguë et comportement.

Nous avons utilisé des souris mâles ($CD_1$ Charles River) d'un poids de 22-23 g à jeûn depuis 16 heures. Les produits leur furent administrés normalement par voie orale aux doses de 1000 - 500 - 250 mg/kg.

L'effet des produits sur le comportement des animaux fut évalué suivant la méthode décrite par Irvin (Psychopharmacologia 13, 222-257, 1968) durant les 8 premières heures et à la 24ème heure.

La mortalité fut relevée pendant les 7 jours suivant le traitement.

La $DL_{50}$ a ainsi été trouvée supérieure à 1000 mg/kg sur les produits des exemples 1 à 3, 5, 7 à 10, 14 à 16 et 34 à 36.

### Apprentissage et mémorisation.

Nous avons utilisé des souris mâles ($CD_1$ Charles River) d'un poids de 25-30 g. Les animaux sont placés dans la partie lumineuse d'un box à deux compartiments communicants par une ouverture (F. Barzaghi et G. Giuliani, Brit. J. Pharmacol. en cours de publication).

A l'instant où la souris passe du compartiment lumineux au compartiment obscur, l'ouverture se ferme et elle est immédiatement punie par une décharge électrique aux pattes. L'animal soumis à cette procédure apprend à mémoriser la punition. En fait, si on le remet dans le compartiment lumineux, il évitera ainsi de franchir l'ouverture et de rentrer dans le compartiment obscur.

Pour induire une amnésie rétrograde, les animaux sont soumis immédiatement après l'apprentissage à un électrochoc. Après l'électrochoc, les produits sont adminitrés par voie orale aux doses de 6,25; 12,5; 25; 50; 100; 200 et 400 mg/kg.

Nous avons utilisé de 10 à 50 animaux par dose.

L'effet antiamnésique des produits est évalué 3 heures après le traitement, en utilisant le même protocole que celui utilisé pour l'acquisition.

Le temps mis par l'animal pour retourner dans la chambre obscure (temps limite 180 secondes) est utilisé comme paramètre d'évaluation.

Dans les mêmes conditions expérimentales, les animaux témoins entrent avec un laps de temps de 40-50 secondes.

Les produits actifs sont ceux qui provoquent une augmentation significative du temps de latence avec une courbe dose-réponse en forme de cloche.

Les résultats sont exprimés en pourcentages d'augmentation du temps de latence, par rapport aux témoins correspondants.

Les résultats sont les suivants :

| Pourcentage d'augmentation du temps de latence par rapport aux témoins | | | | | |
|---|---|---|---|---|---|
| Produit de l'exemple | DOSE : mg/kg : os | | | | |
| | 200 | 100 | 50 | 25 | 12,5 |
| 1 | +109* | +132* | +118* | +95* | +79* |
| 2 | + 23 | + 64* | + 25 | +22 | - |
| 3 | + 7 | + 58* | + 2 | + 5 | 0 |
| 5 | + 75* | + 44 | 0 | +11 | +28 |
| 7 | +108* | + 52* | + 31 | +27 | + 6 |
| 8 | + 75* | + 53* | + 41 | 0 | +24 |
| 9 | + 91* | + 16 | + 30 | +25 | +23 |
| 10 | + 65* | + 18 | + 21 | 0 | +14 |
| 12 | + 59* | + 21 | + 8 | 0 | 0 |
| 14 | + 98* | + 99* | + 47* | +42 | 0 |
| 48 | 0 | + 72* | + 28 | +23 | +11 |
| 15 | + 65* | + 39 | 0 | +14 | 0 |
| 16 | + 47* | + 23 | + 20 | 0 | 0 |
| 31 | + 75* | + 79* | + 65* | +31 | 0 |
| 34 | +105* | + 69* | + 23 | 0 | 0 |
| 35 | + 65* | + 72* | + 95* | +54* | + 4 |
| 37 | +198* | + 95* | + 82* | +34 | |
| 36 | + 82* | + 72* | + 98* | +50* | + 7 |
| 18 | + 88* | + 49 | + 36 | +21 | |
| Pyracétam | 20 | 48* | 10 | 19 | - |
| Aniracétam | 32 | 88* | 77* | 39 | - |

* Valeurs notablement différentes par rapport aux témoins.

Les produits des exemples 1 à 3, 5, 7 à 10, 12, 14, 16, 18, 31, 34 à 37 et 48 ont montré une activité antiamnésique à des doses comprises entre 25 et 200 mg/kg.

Les produits des exemples 1, 14, 35, 36 et 37, en particulier, ont induit une amélioration très notable de la performance des animaux dans une vaste échelle de doses.

## Revendications

**Revendications pour les Etats contractants suivants : BE, CH, DE, FR, GB, IT, LI, LU, NL, SE**

**1.** Les composés répondant à la formule générale (I) :

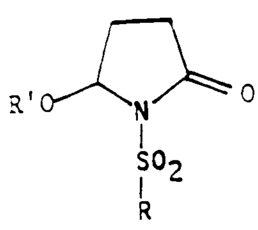

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical furyle, pyrannyle, pyridyle, benzofurannyle, isobenzofurannyle, chromanyle, isochromanyle, chroményle, xanthényle, phénoxathiinyle, oxazolyle, isoxazolyle, furazannyle, phénoxazinyle, thiéno[2,3-b] furannyle, 2H-furo[3,2-b]pyrannyle, benzoxazolyle ou morpholinyle, R étant éventuellement substitué par un ou plusieurs substituants choisis dans le groupe constitué par le radical hydroxyle, le radical acétoxy, le radical méthoxy ou le radical benzyloxy, le radical méthyle, éthyle, propyle ou isopropyle, le radical éthényle ou le radical éthynyle, le fluor, le

chlore, le brome, les groupements $CF_3$, $SCF_3$, $OCF_3$, $NO_2$, $NH_2$ ou C≡N, le radical phényle, les groupements acyles et alcoxycarbonyles renfermant de 2 à 8 atomes de carbone et les groupements alcoylsulfonyles renfermant de l à 6 atomes de carbone.

**2.** Les composés de formule (I) tels que définis à la revendication 1, dans lesquels R représente un radical phényle éventuellement substitué par un radical méthoxy.

**3.** Les composés de formule (I) tels que définis à la revendication 1 ou 2, dans lesquels R' représente un radical alcoyle linéaire, ramifié ou cyclique, renfermant jusqu'à 8 atomes de carbone.

**4.** Les composés tels que définis à la revendication 3, dans lesquels R' représente un radical éthyle.

**5.** Les composés de formule (I) tels que définis à la revendication 1, dont les noms suivent :
- la 1-(benzènesulfonyl) 2-oxo 5-éthoxy pyrrolidine,
- la 1-(4-nitro benzènesulfonyl) 2-oxo 5-éthoxy pyrrolidine,
- la 1-(benzènesulfonyl) 2-oxo 5-isopropoxy pyrrolidine,
- la 1-(benzènesulfonyl) 2-oxo 5-propoxy pyrrolidine.

**6.** A titre de médicaments, les composés tels que définis à l'une quelconque des revendications 1 à 4.

**7.** A titre de médicaments, les composés définis à la revendication 5.

**8.** Les compositions pharmaceutiques renfermant comme principe actif au moins un médicament défini à la revendication 6 ou 7.

**9.** Procédé de préparation des composés de formule (I) tels que définis à l'une quelconque des revendications 1 à 5, caractérisé en ce que l'on soumet un composé de formule (II) :

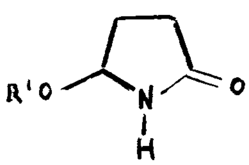

(II)

dans laquelle R' conserve la même signification que dans la revendication 1, à l'action d'un compose de formule (III) :

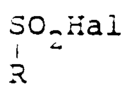

(III)

dans laquelle Hal représente un atome de chlore ou de brome et R conserve la même signification que dans la revendication 1, pour obtenir le composé de formule (I) correspondant.

**10.** Procédé selon la revendication 9, pour la préparation des composés de formule (I) dans laquelle R représente un radical phényle ou naphtyle substitué par un ou plusieurs radicaux amino ou hydroxy, caractérisé en ce que l'on réduit les composés de formule (I) correspondants dans laquelle R représente respectivement un ou plusieurs radicaux nitro, méthoxy ou benzyloxy.

**11.** Procédé de préparation des composés de formule (I), tels que définis à la revendication 1, dans lesquels R est défini comme à la revendication 1 et R' représente un atome d'hydrogène ou un radical acyle renfermant de 1 à 6 atomes de carbone, caractérisé en ce que l'on soumet un halogénure de 4-penténoyle de formule (IV) :

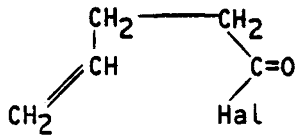

(IV)

dans laquelle Hal représente un atome d'halogène, en présence d'un agent de condensation, à l'action d'un composé de formule (V) :

$H_2N-SO_2-R$ (v)

pour obtenir un composé de formule (VI) :

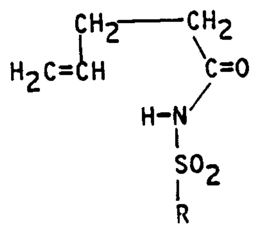

(VI)

que l'on cyclise pour obtenir un composé de formule (I) correspondant, dans laquelle R' représente un atome d'hydrogène :

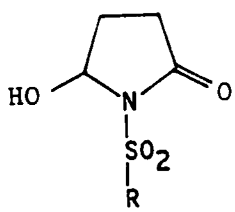

(I avec R' = H)

que l'on acyle, si désiré, pour obtenir un composé de formule (I) correspondant, dans laquelle R' représente un radical acyle :

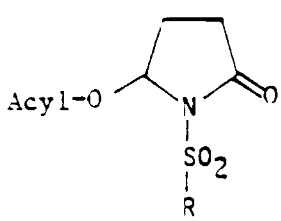

(I avec R' = acyle)

**12.** Les composés suivants :
la 5-éthoxy-1-[(2-thiényl) sulfonyl]-2-pyrrolidinone
la 5-éthoxy-1-[(3-thiényl) sulfonyl]-2-pyrrolidinone.

**Revendications pour les Etats contractants suivants : AT, ES**

1. Procédé de préparation des composés répondant à la formule générale (I) :

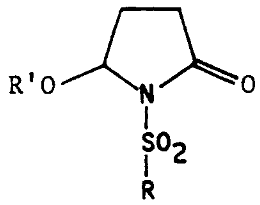

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical furyle, pyrannyle, pyridyle, benzofurannyle, isobenzofurannyle, chromanyle, isochromanyle, chroményle, xanthényle, phénoxathiinyle, oxazolyle, isoxazolyle, furazannyle, phénoxazinyle, thiéno[2,3-b] furannyle, 2H-furo(3,2-b]pyrannyle, benzoxazolyle ou morpholinyle, R étant éventuellement substitué par un ou plusieurs substituants choisis dans le groupe constitué par le radical hydroxyle, le radical acétoxy, le radical méthoxy ou le radical benzyloxy, le radical méthyle, éthyle, propyle ou isopropyle, le radical éthényle ou le radical éthynyle, le fluor, le chlore, le brome, les groupements $CF_3$, $SCF_3$, $OCF_3$, $NO_2$, $NH_2$ ou C≡N, le radical phényle, les groupements acyles et alcoxycarbonyles renfermant de 2 à 8 atomes de carbone et les groupements alcoylsulfonyles renfermant de I à 6 atomes de carbone, caractérisé en ce que l'on soumet un composé de formule (II) :

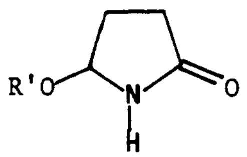

(II)

dans laquelle R' conserve la même signification que précédemment, à l'action d'un composé de formule (III) :

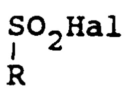

(III)

dans laquelle Hal représente un atome de chlore ou de brome et R conserve la même signification que précédemment, pour obtenir le composé de formule (I) correspondant.

2. Procédé selon la revendication 1, pour la préparation des composés répondant à la formule générale (I') :

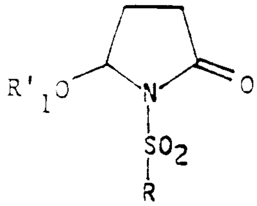

(I')

dans laquelle $R'_1$ représente un atome d'hydrogène, un radical alcoyle linéaire, ramifié ou cyclique

renfermant jusqu'à 8 atomes de carbone ou un radical acyle renfermant de 1 à 6 atomes de carbone et R est défini comme à la revendication 1, caractérisé en ce que l'on utilise au départ un composé de formule (II) dans laquelle R' a les valeurs de R'1 indiquées ci-dessus.

**3.** Procédé selon la revendication 1, pour la préparation des composés de formule (I) dans laquelle R représente un radical phényle ou naphtyle substitué par un ou plusieurs radicaux amino ou hydroxy, caractérisé en ce que l'on réduit les composés de formule (I) correspondants dans laquelle R représente respectivement un ou plusieurs radicaux nitro, méthoxy ou benzyloxy.

**4.** Procédé de préparation des composés de formule (I), tels que définis à la revendication 1, dans lesquels R est défini comme à la revendication 1 et R' représente un atome d'hydrogène ou un radical acyle renfermant de 1 à 6 atomes de carbone, caractérisé en ce que l'on soumet un halogénure de 4-pentényle de formule (IV) :

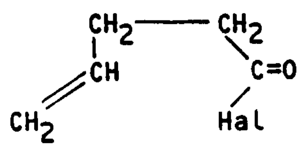

(IV)

dans laquelle Hal représente un atome d'halogène, en présence d'un agent de condensation, à l'action d'un composé de formule (V) :

$H_2N\text{-}SO_2\text{-}R$ (v)

pour obtenir un composé de formule (VI) :

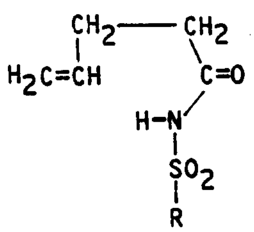

(VI)

que l'on cyclise pour obtenir un composé de formule (I) correspondant, dans laquelle R' représente un atome d'hydrogène :

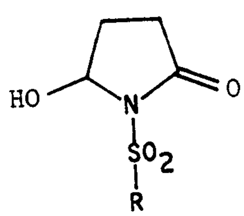

(I avec R' = H)

que l'on acyle, si désiré, pour obtenir un composé de formule (I) correspondant, dans laquelle R' représente un radical acyle :

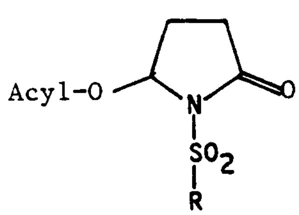

(I avec R' = acyle)

**5.** Procédé selon l'une quelconque des revendications 1 à 4, caractérisé en ce que l'on utilise au départ un composé de formule (III) ou (V) dans laquelle R représente un radical phényle, éventuellement substitué par radical méthoxy.

**6.** Procédé selon l'une quelconque des revendications 1 à 3, caractérisé en ce que l'on utilise au départ un composé de formule (II) dans laquelle R' représente un radical alcoyle renfermant jusqu'à 8 atomes de carbone.

**7.** Procédé selon la revendication 6, caractérisé en ce que R' représente un radical éthyle.

**8.** Procédé selon la revendication 2, caractérisé en ce que l'on utilise au départ un composé de formule (II) dans laquelle R' représente un radical éthyle et un composé de formule (III) dans laquelle R représente un radical phényle.

**9.** Procédé selon la revendication 2, caractérisé en ce que l'on utilise au départ un composé de formule (II) dans laquelle R' représente un radical éthyle, isopropyle ou propyle et un composé de formule (III) dans laquelle R représente un radical phényle ou un radical 4-nitro phényle.

**10.** Procédé selon la revendication 1, caractérisé en ce que le produit préparé est la 5-éthoxy-1-[(2-thiényl) sulfonyl]-2-pyrrolidinone ou la 5-éthoxy-1-[(3-thiényl) sulfonyl]-2-pyrrolidinone.

**Revendications pour l'Etat contractant suivant : GR**

**1.** Procédé de préparation des composés répondant à la formule générale (I) :

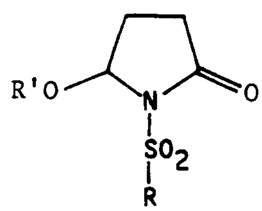

(I)

dans laquelle R' représente un atome d'hydrogène, un radical alcoyle, linéaire, ramifié ou cyclique renfermant jusqu'à 8 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou naphtyle ou un radical furyle, pyrannyle, pyridyle, benzofurannyle, isobenzofurannyle, chromanyle, isochromanyle, chroményle, xanthényle, phénoxathiinyle, oxazolyle, isoxazolyle, furazannyle, phénoxazinyle, thiéno[2,3-b] furannyle, 2H-furo[3,2-b]pyrannyle, benzoxazolyle ou morpholinyle, R étant éventuellement substitué par un ou plusieurs substituants choisis dans le groupe constitué par le radical hydroxyle, le radical acétoxy, le radical méthoxy ou le radical benzyloxy, le radical méthyle, éthyle, propyle ou isopropyle, le radical éthényle ou le radical éthynyle, le fluor, le chlore, le brome, les groupements $CF_3$, $SCF_3$, $OCF_3$, $NO_2$, $NH_2$ ou C≡N, le radical phényle, les groupements acyles et alcoxycarbonyles renfermant de 2 à 8 atomes de carbone et les groupements alcoylsulfonyles renfermant de 1 à 6 atomes de carbone, caractérisé en ce que l'on soumet un composé de formule (II) :

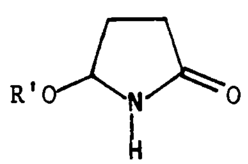

(II)

dans laquelle R' conserve la même signification que précédemment, à l'action d'un composé de formule (III) :

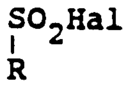

(III)

dans laquelle Hal représente un atome de chlore ou de brome et R conserve la même signification que précédemment, pour obtenir le composé de formule (I) correspondant.

2. Procédé selon la revendication 1, pour la préparation des composés répondant à la formule générale (I') :

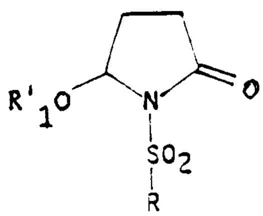

(I')

dans laquelle $R'_1$ représente un atome d'hydrogène, un radical alcoyle linéaire, ramifié ou cyclique renfermant jusqu'à 8 atomes de carbone ou un radical acyle renfermant de 1 à 6 atomes de carbone et R est défini comme à la revendication 1, caractérisé en ce que l'on utilise au départ un composé de formule (II) dans laquelle R' a les valeurs de R'1 indiquées ci-dessus.

3. Procédé selon la revendication 1, pour la préparation des composés de formule (I) dans laquelle R représente un radical phényle ou naphtyle substitué par un ou plusieurs radicaux amino ou hydroxy, caractérisé en ce que l'on réduit les composés de formule (I) correspondants dans laquelle A représente respectivement un ou plusieurs radicaux nitro, méthoxy ou benzyloxy.

4. Procédé de préparation des composés de formule (I), tels que définis à la revendication 1, dans lesquels R est défini comme à la revendication 1 et R' représente un atome d'hydrogène ou un radical acyle renfermant de 1 à 6 atomes de carbone, caractérisé en ce que l'on soumet un halogénure de 4-penténoyle de formule (IV) :

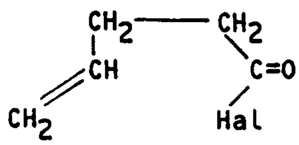

(IV)

dans laquelle Hal représente un atome d'halogène, en présence d'un agent de condensation, à l'action d'un composé de formule (V) :

$H_2N\text{-}SO_2\text{-}R$ (V)

pour obtenir un composé de formule (VI) :

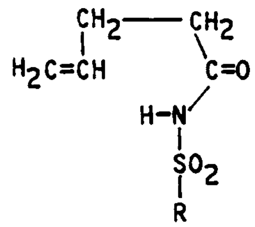

(VI)

que l'on cyclise pour obtenir un composé de formule (I) correspondant, dans laquelle R' représente un atome d'hydrogène :

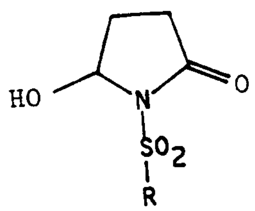

(I avec R' = H)

que l'on acyle, si désiré, pour obtenir un composé de formule (I) correspondant, dans laquelle R' représente un radical acyle :

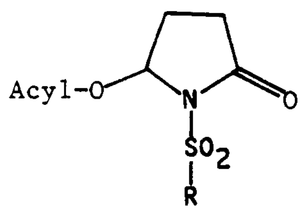

(I avec R' = acyle)

**5.** Procédé selon l'une quelconque des revendications 1 à 4, caractérisé en ce que l'on utilise au départ un composé de formule (III) ou (V) dans laquelle R représente un radical phényle, éventuellement substitué par radical méthoxy.

**6.** Procédé selon l'une quelconque des revendications 1 à 3, caractérisé en ce que l'on utilise au départ un composé de formule (II) dans laquelle R' représente un radical alcoyle renfermant jusqu'à 8 atomes de carbone.

**7.** Procédé selon la revendication 6, caractérisé en ce que R' représente un radical éthyle.

**8.** Procédé selon la revendication 2, caractérisé en ce que l'on utilise au départ un composé de formule (II) dans laquelle R' représente un radical éthyle et un composé de formule (III) dans laquelle R représente un radical phényle.

**9.** Procédé selon la revendication 2, caractérisé en ce que l'on utilise au départ un composé de formule (II) dans laquelle R' représente un radical éthyle, isopropyle ou propyle et un composé de formule (III) dans laquelle R représente un radical phényle ou un radical 4-nitro phényle.

**10.** Procédé de préparation de compositions pharmaceutiques, caractérisé en ce que l'on met, à titre de principe actif, l'un au moins des produits de formule (I), tels que définis à la revendication 1, sous une forme destinée à cet usage.

**11.** Procédé de préparation de compositions pharmaceutiques, caractérisé en ce que l'on met, à titre de principe actif, l'un au moins des produits de formule (I'), tels que définis à la revendication 2, sous une forme destinée à cet usage.

**12.** Procédé selon la revendication 11, caractérisé en ce que, dans la formule (I'), R représente un radical phényle éventuellement substitué par l'un des substituants définis à la revendication 1 et $R'_1$ représente un radical alcoyle renfermant jusqu'à 8 atomes de carbone.

**13.** Procédé selon la revendication 11, caractérisé en ce que le principe actif est la 1-(benzènesulfonyl) 2-oxo 5-éthoxy pyrrolidine.

**14.** Procédé selon la revendication 1 caractérisé en ce que le produit préparé est la 5-éthoxy-1-[(2-thiényl) sulfonyl]-2-pyrrolidinone ou la 5-éthoxy-1-[(3-thiényl) sulfonyl]-2-pyrrolidinone.

## Claims

### Claims for the following Contracting States : BE, CH, DE, FR, GB, IT, LI, LU, NL, SE

**1.** The compounds corresponding to general formula (I):

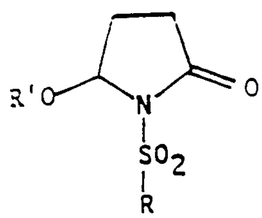

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or naphthyl radical or one of the following radicals: furyl, pyrannyl, pyridyl, benzofurannyl, isobenzofurannyl, chromanyl, isochromanyl, chromenyl, xanthenyl, phenoxathiinyl, oxazolyl, isoxazolyl, furazannyl, phenoxazinyl, thieno-[2,3-b]furannyl, 2H-furo[3,2-b]pyrannyl, benzoxasolyl or morpholinyl, R being optionally substituted by one or more substituents chosen from the group constituted by the following radicals: hydroxyl, acetoxy, methoxy or benzyloxy, methyl, ethyl, propyl or isopropyl, ethenyl or ethynyl, fluorine, chlorine, bromine, or the following groups: $CF_3$, $SCF_3$, $OCF_3$, $NO_2$, $NH_2$ or C≡N, the phenyl radicals, acyl and alkoxycarbonyl groups containing 2 to 8 carbon atoms and alkylsulphonyl groups containing 1 to 6 carbon atoms.

**2.** The compounds of formula (I) as defined in claim 1, in which R represents a phenyl radical optionally substituted by a methoxy radical.

**3.** The compounds of formula (I) as defined in claim 1 or 2, in which R' represents a linear, branched or cyclic alkyl radical containing up to 8 carbon atoms.

**4.** The compounds as defined in claim 3, in which R' represents an ethyl radical.

**5.** The compounds of formula (I) as defined in claim 1, the names of which follow:
- 1-(benzenesulphonyl)-2-oxo-5-ethoxy pyrrolidine,
- 1-(4-nitro-benzenesulphonyl)-2-oxo-5-ethoxy pyrrolidine,
- 1-(benzenesulphonyl)-2-oxo-5-isopropoxy pyrrolidine,
- 1-(benzenesulphonyl)-2-oxo-5-propoxy pyrrolidine.

**6.** As medicaments, the compounds as defined in any one of claims 1 to 4.

**7.** As medicaments, the compounds defined in claim 5.

**8.** The pharmaceutical compositions containing as active ingredient at least one medicament defined in claim 6 or 7.

**9.** Preparation process for the compounds of formula (I) as defined in any one of claims 1 to 5, characterized in that a compound of formula (II):

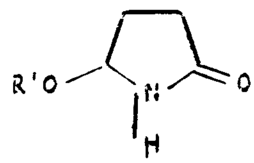

(II)

in which R' retains the same meaning as in claim 1, is subjected to the action of a compound of formula (III):

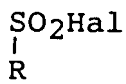

(III)

in which Hal represents a chlorine or bromine atom and R retains the same meaning as in claim 1, in order to obtain the corresponding compound of formula (I).

**10.** Process according to claim 9, for the preparation of compounds of formula (I) in which R represents a phenyl or naphthyl radical substituted by one or more amino or hydroxy radicals, characterized in that the corresponding compounds of formula (I) in which R represents respectively one or more nitro, methoxy or benzyloxy radicals are reduced.

**11.** Preparation process for the compounds of formula (I), as defined in claim 1, in which R is defined as in claim 1 and R' represents a hydrogen atom or an acyl radical containing 1 to 6 carbon atoms, characterized in that a 4-pentenoyl halide of formula (IV):

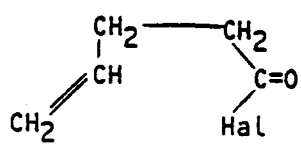

(IV)

in which Hal represents a halogen atom, is subjected, in the presence of a condensation agent, to the action of a compound of formula (V):

$H_2N-SO_2-R$ (V)

in order to obtain a compound of formula (VI):

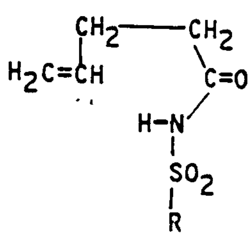

(VI)

which is cyclized in order to obtain the corresponding compound of formula (I), in which R' represents a hydrogen atom:

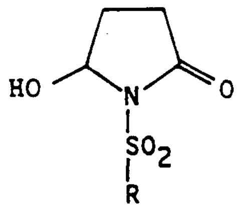

(I with R'= H)

which is acylated, if desired, in order to obtain a corresponding compound of formula (I), in which R' represents an acyl radical:

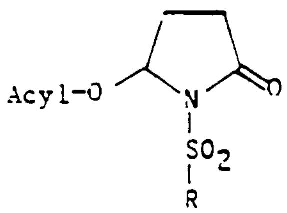

(I with R'= acyl)

**12.** The following compounds:
5-ethoxy-1-[(2-thienyl)-sulphonyl]-2-pyrrolidinone,
5-ethoxy-1-[(3-thienyl)-sulphonyl]-2-pyrrolidinone.

**Claims for the following Contracting States : AT, ES**

**1.** Preparation process for the compounds corresponding to general formula (I):

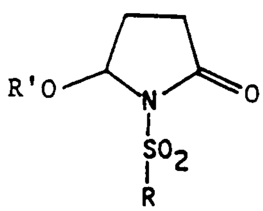

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or naphthyl radical or one of the following radicals: furyl, pyrannyl, pyridyl, benzofurannyl, isobenzofurannyl, chromanyl, isochromanyl, chromenyl, xanthenyl, phenoxathiinyl, oxazolyl, isoxazolyl, furazannyl, phenoxazinyl, thieno-[2,3-b]furannyl, 2H-furo[3,2-b]pyrannyl, benzoxazolyl or morpholinyl, R being optionally substituted by one or more substituents chosen from the group constituted by the following radicals: hydroxyl, acetoxy methoxy or benzyloxy, methyl, ethyl, propyl or isopropyl, ethenyl or ethynyl, fluorine, chlorine, bromine, or the following groups: $CF_3$, $SCF_3$, $OCF_3$, $NO_2$, $NH_2$ or C≡N, the phenyl radical acyl and alkoxycarbonyl groups containing 2 to 8 carbon atoms and alkylsulphonyl groups containing 1 to 6 carbon atoms, characterized in that a compound of formula (II):

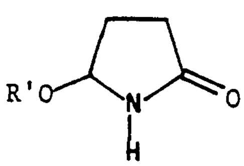

(II)

in which R' retains the same meaning as previously, is subjected to the action of a compound of formula (III):

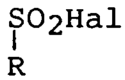

(III)

in which Hal represents a chlorine or bromine atom and R retains the same meaning as previously, in order to obtain the corresponding compound of formula (I).

2. Process according to claim 1, for the preparation of compounds corresponding to general formula (I'):

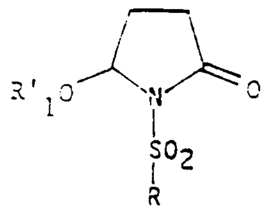

(I')

in which $R'_1$ represents a hydrogen atom, a linear, branched or cyclic alkyl radical containing up to 8 carbon atoms or an acyl radical containing 1 to 6 carbon atoms and R is as defined in claim 1, characterized in that a compound of formula (II) is used at the start in which R' has the values of $R'_1$ indicated above.

3. Process according to claim 1, for the preparation of compounds of formula (I) in which R represents a phenyl or naphthyl radical substituted by one or more amino or hydroxy radicals, characterized in that the corresponding compounds of formula (I) in which R represents respectively one or more nitro, methoxy or benzyloxy radicals, are reduced.

4. Preparation process for the compounds of formula (I), as defined in claim 1, in which R is defined as in claim 1 and R' represents a hydrogen atom or an acyl radical containing 1 to 6 carbon atoms, characterized in that a 4-pentenoyl halide of formula (IV):

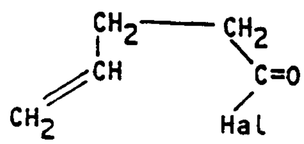

(IV)

in which Hal represents a halogen atom is subjected, in the presence of a condensation agent, to the action of a compound of formula (V):

$H_2N\text{-}SO_2\text{-}R$ (V)

in order to obtain a compound of formula (VI):

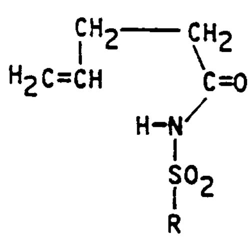

(VI)

which is cyclized in order to obtain the corresponding compound of formula (I), in which R' represents a hydrogen atom:

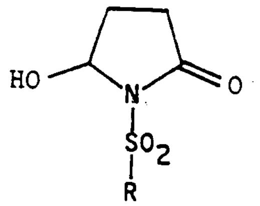

(I with R'= H)

which is acylated, if desired, in order to obtain a corresponding compound of formula (I), in which R' represents an acyl radical:

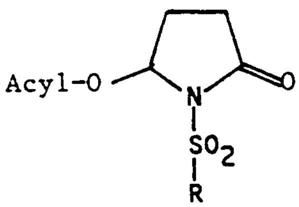

(I with R'= acyl)

**5.** Process according to any one of claims 1 to 4, characterized in that a compound of formula (III) or (V) is used at the start in which R represents a phenyl radical optionally substituted by a methoxy radical.

**6.** Process according to any one of claims 1 to 3, characterized in that a compound of formula (II) is used at the start in which R' represents an alkyl radical containing up to 8 carbon atoms.

**7.** Process according to claim 6, characterized in that R' represents an ethyl radical.

**8.** Process according to claim 2, characterized in that a compound of formula (II) in which R' represents an ethyl radical and a compound of formula (III) in which R represents a phenyl radical are used at the start.

**9.** Process according to claim 2, characterized in that a compound of formula (II) in which R' represents an ethyl, isopropyl or propyl radical and a compound of formula (III) in which R represents a phenyl or a 4-nitro phenyl radical are used at the start.

**10.** Process according to claim 1, characterized in that the product prepared is 5-ethoxy-1-[(2-thienyl)-sulphonyl]-2-pyrrolidinone or 5-ethoxy-1-[(3-thienyl)-sulphonyl]-2-pyrrolidinone.

**Claims for the following Contracting State : GR**

**1.** Preparation process for the compounds corresponding to general formula (I):

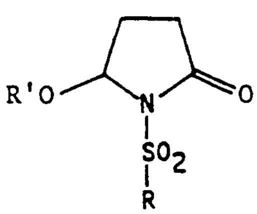

(I)

in which R' represents a hydrogen atom, a linear, branched or cyclic alkyl radical containing up to 8 carbon atoms, an alkenyl radical containing 2 to 8 carbon atoms, an acyl radical containing 1 to 6 carbon atoms or a benzyl or phenethyl radical and R represents a phenyl or naphthyl radical or on of the following radicals: furyl, pyrannyl, pyridyl, benzofurannyl, isobenzofurannyl, chromanyl, isochromanyl, chromenyl, xanthenyl, phenoxathiinyl, oxazolyl, isoxazolyl, furazannyl, phenoxazinyl, thieno-[2,3-b]furannyl, 2H-furo[3,23-b]pyrannyl, bensoxazolyl or morpholinyl, R being optionally substituted by

one or more substituents chosen from the group constituted by the following radicals: hydroxyl, acetoxy, methoxy or benzyloxy, methyl, ethyl, propyl or isopropyl, ethenyl or ethynyl, fluorine, chlorine, bromine, or the following groups: $CF_3$, $SCF_3$, $OCF_3$, $NO_2$, $NH_2$ or C≡N, the phenyl radical, acyl and alkoxycarbonyl groups containing 2 to 8 carbon atoms and alkylsulphonyl groups containing 1 to 6 carbon atoms, characterized in that a compound of formula (II):

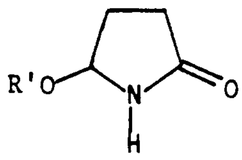

(II)

in which R' retains the same meaning as previously, is subjected to the action of a compound of formula (III):

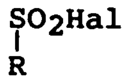

(III)

in which Hal represents a chlorine or bromine atom and R retains the same meaning as previously.

2. Process according to claim 1, for the preparation of compounds corresponding to general formula (I'):

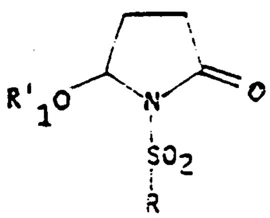

(I')

in which $R'_1$ represents a hydrogen atom, a linear, branched or cyclic alkyl radical containing up to 8 carbon atoms or an acyl radical containing 1 to 6 carbon atoms and R is as defined in claim 1, characterized in that a compound of formula (II) is used at the start in which R' has the values of $R'_1$ indicated above.

3. Process according to claim 1, for the preparation of compounds of formula (I) in which R represents a phenyl or naphthyl radical substituted by one or more amino or hydroxy radicals, characterized in that the corresponding compounds of formula (I) in which R represents respectively one or more nitro, methoxy or benzyloxy radicals, are reduced.

4. Preparation process for the compounds of formula (I), as defined in claim 1, in which R is defined as in claim 1 and R' represents a hydrogen atom or an acyl radical containing 1 to 6 carbon atoms, characterized in that a 4-pentenoyl halide of formula (IV):

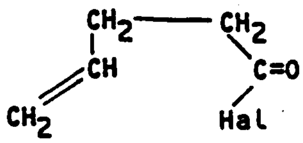

(IV)

in which Hal represents a halogen atom is subjected, in the presence of a condensation agent, to the action of a compound of formula (V):

$H_2N-SO_2-R$ (V)

in order to obtain a compound of formula (VI):

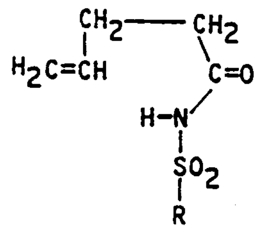

(VI)

which is cyclized in order to obtain the corresponding compound of formula (I), in which R' represents a hydrogen atom:

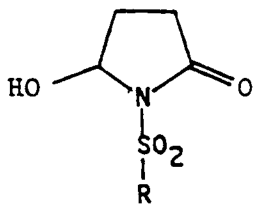

(I with R'= H)

which is acylated, if desired, in order to obtain a corresponding compound of formula (I), in which R' represents an acyl radical:

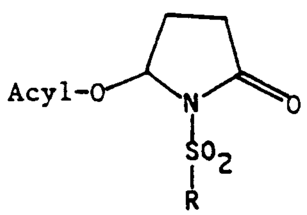

(I with R'= acyl)

**5.** Process according to any one of claims 1 to 4, characterized in that a compound of formula (III) or (V) is used at the start in which R represents a phenyl radical optionally substituted by a methoxy radical.

**6.** Process according to any one of claims 1 to 3, characterized in that a compound of formula (II) is used at the start in which R' represents an alkyl radical containing up to 8 carbon atoms.

**7.** Process according to claim 6, characterized in that R' represents an ethyl radical.

**8.** Process according to claim 2, characterized in that a compound of formula (II) in which R' represents an ethyl radical and a compound of formula (III) in which R represents a phenyl radical are used at the start.

**9.** Process according to claim 2, characterized in that a compound of formula (II) in which R' represents an ethyl, isopropyl or propyl radical and a compound of formula (III) in which R represents a phenyl or a 4-nitro phenyl radical are used at the start.

**10.** Preparation process for pharmaceutical compositions, characterized in that at least one of the products of formula (I) as defined in claim 1, is used as the active ingredient in a form intended for this use.

**11.** Preparation process for pharmaceutical compositions, characterized in that at least one of the products of formula (I') as defined in claim 2 is used as the active ingredient in a form intended for this use.

**12.** Process according to claim 11, characterized in that in formula (I') R represents a phenyl radical optionally substituted by one of the substituents defined in claim 1 and $R'_1$ represents an alkyl radical containing up to 8 carbon atoms.

**13.** Process according to claim 11, characterized in that the active ingredient is 1-(benzenesulphonyl)-2-oxo-5-ethoxy pyrrolidine.

**14.** Process according to claim 1, characterized in that the product prepared is 5-ethoxy-1-[(2-thienyl)-sulphonyl]-2-pyrrolidinone or 5-ethoxy-1-[(3-thienyl)-sulphonyl]-2-pyrrolidinone.

## Patentansprüche

### Patentansprüche für folgende Vertragsstaaten : BE, CH, DE, FR, GB, IT, LI, LU, NL, SE

**1.** Verbindungen der allgemeinen Formel (I):

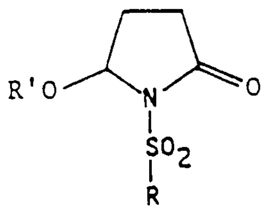

(I)

worin R' ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest bedeutet und R fur einen Phenyl- oder Naphthylrest oder einen Furyl-, Pyranyl-, Pyridyl-, Benzofuranyl-, Isobenzofuranyl-, Chromanyl-, Isochromanyl-, Chromenyl-, Xanthenyl-, Phenoxathiinyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest steht, wobei R gegebenenfalls durch einen oder mehrere Substituenten, ausgewählt unter dem Hydroxylrest, der Acetoxygruppe, dem Methoxyrest oder dem Benzyloxyrest, der Methyl-, Ethyl-, Propyl- oder Isopropylgruppe, dem Ethenylrest oder dem Ethinylrest, Fluor, Chlor, Brom, den Gruppen $CF_3$, $SCF_3$, $OCF_3$, $NO_2$, $NH_2$ oder C≡H, dem Phenylrest, den Acyl- und den Alkoxycarbonylgruppen mit 2 bis 8 Kohlenstoffatomen und den Alklsulfonylgruppen mit 1 bis 6 Kohlenstoffatomen, substituiert ist.

**2.** Verbindungen der Formel (I), wie in Anspruch 1 definiert, worin R für einen gegebenenfalls durch einen Methoxyrest substituierten Phenylrest steht.

**3.** Verbindungen der Formel (I), wie in Anspruch 1 oder 2 definiert, worin R' für einen linearen, verzweigten oder cyclischen Alkylrest mit bis zu 8 Kohlenstoffatomen steht.

**4.** Verbindungen gemäß Anspruch 3, worin R' für einen Ethylrest steht.

**5.** Verbindungen der Formel (I), wie in Anspruch 1 definiert, mit den folgenden Bezeichnungen:
1-(Benzolsulfonyl)-2-oxo-5-ethoxypyrrolidin,
1-(4-Nitrobenzolsulfonyl)-2-oxo-5-ethoxypyrrolidin,
1-(Benzolsulfonyl)-2-oxo-5-isopropoxypyrrolidin,
1-(Benzolsulfonyl)-2-oxo-5-propoxypyrrolidin.

**6.** Als Arzneimittel die Verbindungen, wie in einem der Ansprüche 1 bis 4 definiert.

**7.** Als Arzneimittel die Verbindungen, wie in Anspruch 5 definiert.

**8.** Pharmazeutische Zusammensetzungen, enthaltend als Wirkstoff zumindest ein Arzneimittel, wie in Anspruch 6 oder 7 definiert.

**9.** Verfahren zur Herstellung der Verbindungen der Formel (I), wie in einem der Ansprüche 1 bis 5 definiert, dadurch gekennzeichnet, daß man eine Verbindung der Formel (II)

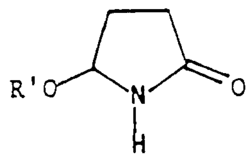

(II)

worin R' wie in Anspruch 1 definiert ist, der Einwirkung einer Verbindung der Formel (III)

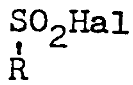

(III)

worin Hai für ein Chlor- oder Bromatom steht und R wie in Anspruch 1 definiert ist, unterzieht, um die entsprechende Verbindung der Formel (I) zu erhalten.

**10.** Verfahren gemäß Anspruch 9 zur Herstellung von Verbindungen der Formel (I), worin R für einen Phenyloder Naphthylrest steht, der durch einen oder mehrere Amino- oder Hydroxyrestesubstituiert ist, dadurch gekennzeichnet, daß man die entsprechenden Verbindungen der Formel (I), worin R eine oder mehrere Nitro-, Methoxy oder Benzyloxygruppen bedeutet, reduziert.

**11.** Verfahren zur Herstellung der Verbindungen der Formel (I), wie in Anspruch 1 definiert, worin R die in Anspruch 1 angegebene Bedeutung besitzt und R' für ein Wasserstoffatom oder einen Acylrest mit 1 bis 6 Kohlenstoffatomen steht, dadurch gekennzeichnet, daß man ein 4-Pentenoylhalogenid der Formel (IV)

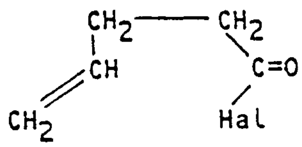

(IV)

worin Hal für ein Halogenatom steht, in Anwesenheit eines Kondensationsmittels der Einwirkung einer Verbindung der Formel (V)

$H_2N\text{-}SO_2\text{-}R$ (v)

unterzieht, um zu einer Verbindung der Formel (VI)

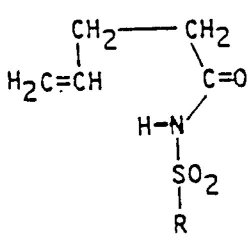

(VI)

zu gelangen, die man cyclisiert, um eine entsprechende Verbindung der Formel (I) zu erhalten, worin R' für ein Wasserstoffatom steht:

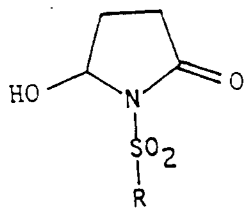

(I mit R' = H)

welche man gewünschtenfalls acyliert, um zu einer entsprechenden Verbindung der Formel (I) zu gelangen, worin R' für einen Acylrest steht:

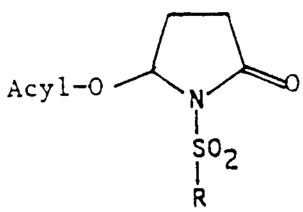

(I mit R' = Acyl).

**12.** Die folgenden Verbindungen:
5-Ethoxy-1-[(2-thienyl)-sulfonyl]-2-pyrrolidinon
5-Ethoxy-1-[(3-thienyl)-sulfonyl]-2-pyrrolidinon.

**Patentansprüche für folgende Vertragsstaaten : AT, ES**

**1.** Verfahren zur Herstellung von Verbindungen der allgemeinen Formel (I)

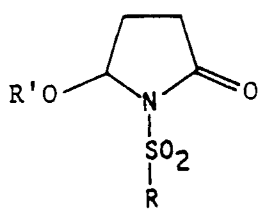

(I)

worin R' ein Wasserstoffatom, einen linearen, verzweigten oder cyclischen Alkylrest mit bis zu 8 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest bedeutet und R für einen Phenyl- oder Naphthylrest oder einen Furyl-, Pyranyl-, Pyridyl-, Benzofuranyl-, Isobenzofuranyl-, Chromanyl-, Isochromanyl-, Chromenyl-, Xanthenyl-, Phenoxathiinyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest steht, wobei R gegebenenfalls durch einen oder mehrere Substituenten, ausgewählt unter dem Hydroxylrest, der Acetoxygruppe, dem Methoxyrest oder dem Benzyloxyrest, der Methyl-, Ethyl-, Propyl- oder Isopropylgruppe, dem Ethenylrest oder dem Ethinylrest, Fluor, Chlor, Brom, den Gruppen $CF_3$, $SCF_3$, $OCF_3$, $NO_2$, $NH_2$ oder C≡N, dem Phenylrest, den Acyl- und den Alkoxycarbonylgruppen mit 2 bis 8 Kohlenstoffatomen und den Alkylsulfonylgruppen mit 1 bis 6 Kohlenstoffatomen, substituiert ist, dadurch **gekennzeich-net,** daß man eine Verbindung der Formel (II)

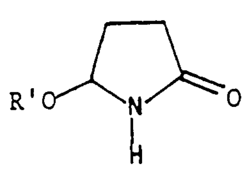

(II)

worin R' die vorstehend angegebene Bedeutung besitzt, der Einwirkung einer Verbindung der Formel (III)

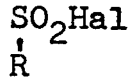

(III)

worin Hal für ein Chlor- oder Bromatom steht und R die vorstehend angegebene Bedeutung besitzt, unterzieht, um die entsprechende Verbindung der Formel (I) zu erhalten.

**2.** Verfahren gemäß Anspruch 1 zur Herstellung von Verbindungen der allgemeinen Formel (I')

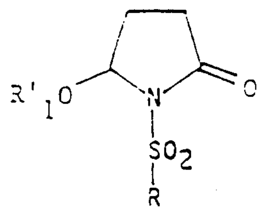

(I')

worin $R'_1$ für ein Wasserstoffatom, einen linearen, verzweigten oder cyclischen Alkylrest mit bis zu 8 Kohlenstoffatomen oder einen Acylrest mit 1 bis 6 Kohlenstoffatomen steht und R wie in Anspruch 1 definiert ist, dadurch gekennzeichnet, daß man von einer Verbindung der Formel (II) ausgeht, worin R' die vorstehend für $R'_1$ angegebenen Bedeutungen besitzt.

**3.** Verfahren gemäß Anspruch 1 zur Herstellung von Verbindungen der Formel (I), worin R für einen Phenyl-oder Naphthylrest steht, der durch einen oder mehrere Amino- oder Hydroxyreste substituiert ist, dadurch gekennzeichnet, daß man die entsprechenden Verbindungen der Formel (I), worin R eine oder mehrere Nitro-, Methoxy- oder Benzyloxygruppen bedeutet, reduziert.

**4.** Verfahren zur Herstellung der Verbindungen der Formel (I), wie in Anspruch 1 definiert, worin R die in Anspruch 1 angegebene Bedeutung besitzt und R' für ein Wasserstoffatom oder einen Acylrest mit 1 bis 6 Kohlenstoffatomen steht, dadurch gekennzeichnet, daß man ein 4-Pentenoylhalogenid der Formel (IV)

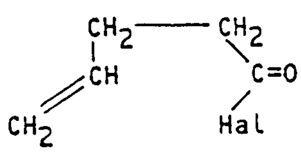

(IV)

worin Hal für ein Halogenatom steht, in Anwesenheit eines Kondensationsmittels der Einwirkung einer Verbindung der Formel (V)

$H_2N\text{-}SO_2\text{-}R$ (v)

unterzieht, um zu einer Verbindung der Formel (VI)

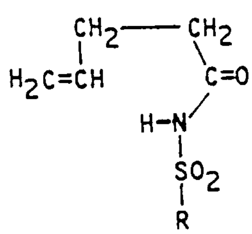

(VI)

zu gelangen, die man cyclisiert, um eine entsprechende Verbindung der Formel (I) zu erhalten, worin R' für ein Wasserstoffatom steht:

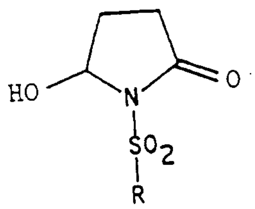

(I mit R' = H)

welche man gewünschtenfalls acyliert, um zu einer entsprechenden Verbindung der Formel (I) zu gelangen, worin R' für einen Acylrest steht:

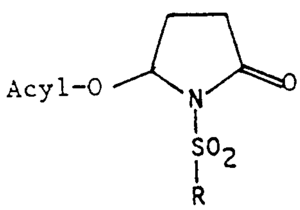

(I mit R' = Acyl).

**5.** Verfahren gemäß einem der Ansprüche 1 bis 4, dadurch gekennzeichnet, daß man von einer Verbindung der Formel (III) oder (V) ausgeht, worin R für eine gegebenenfalls durch einen Methoxyrest substituierte Phenylgruppe steht.

**6.** Verfahren gemäß einem der Ansprüche 1 bis 3, dadurch gekennzeichnet, daß man von einer Verbindung der Formel (II) ausgeht, worin R' für einen Alkylrest mit bis zu 8 Kohlenstoffatomen steht.

**7.** Verfahren gemäß Anspruch 6, dadurch gekennzeichnet, daß R' einen Ethylrest bedeutet.

**8.** Verfahren gemäß Anspruch 2, dadurch gekennzeichnet, daß man von einer Verbindung der Formel (II), worin R' für einen Ethylrest steht, und einer Verbindung der Formel (III), worin R für einen Phenylrest steht, ausgeht.

**9.** Verfahren gemäß Anspruch 2, dadurch gekennzeichnet, daß man von einer Verbindung der Formel (II), worin R' für einen Ethyl-, Isopropyl- oder Propylrest steht, und einer Verbindung der Formel (III), worin R für einen Phenyl- oder einen 4-Nitrophenylrest steht, ausgeht.

**10.** Verfahren gemäß Anspruch 1, dadurch gekennzeichnet, daß das hergestellte Produkt das 5-Ethoxy-1-[-(2-thienyl)-sulfonyl]-2-pyrrolidinon oder das 5-Ethoxy-1-[(3-thienyl)-sulfonyl]-2-pyrrolidinon ist.

**Patentansprüche für folgenden Vertragsstaat : GR**

**1.** Verfahren zur Herstellung von Verbindungen der allgemeinen Formel (I)

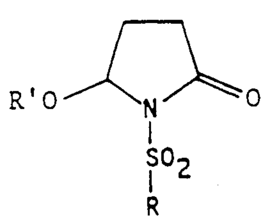

(I)

worin R' ein Wasserstoffatom, einen linearen, verzweigten oder cyclischen Alkylrest mit bis zu 8

Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest bedeutet und R für einen Phenyl- oder Naphthylrest oder einen Furyl-, Pyranyl-, Pyridyl-, Benzofuranyl-, Isobenzofuranyl-, Chromanyl-, Isochromanyl-, Chromenyl-, Xanthenyl-, Phenoxathiinyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest steht, wobei R gegebenenfalls durch einen oder mehrere Substituenten, ausgewählt unter dem Hydroxylrest, der Acetoxygruppe, dem Methoxyrest oder dem Benzyloxyrest, der Methyl-, Ethyl-, Propyl- oder Isopropylgruppe, dem Ethenylrest oder dem Ethinylrest, Fluor, Chlor, Brom, den Gruppen $CF_3$, $SCF_3$, $OCF_3$, $NO_2$, $NH_2$ oder C≡N, dem Phenylrest, den Acyl- und den Alkoxycarbonylgruppen mit 2 bis 8 Kohlenstoffatomen und den Alkylsulfonylgruppen mit 1 bis 6 Kohlenstoffatomen, substituiert ist, da-durch **gekennzeich-net,** daß man eine Verbindung der Formel (II)

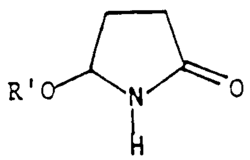

(II)

worin R' die vorstehend angegebene Bedeutung besitzt, der Einwirkung einer Verbindung der Formel (III)

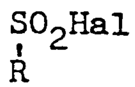

(III)

worin Hal für ein Chlor- oder Bromatom steht und R die vorstehend angegebene Bedeutung besitzt, unterzieht, um die entsprechende Verbindung der Formel (I) zu erhalten.

**2.** Verfahren gemäß Anspruch 1 zur Herstellung von Verbindungen der allgemeinen Formel (I')

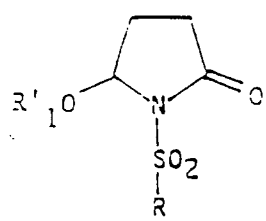

(I')

worin $R'_1$ für ein Wasserstoffatom, einen linearen, verzweigten oder cyclischen Alkylrest mit bis zu 8 Kohlen-stoffatomen oder einen Acylrest mit 1 bis 6 Kohlenstoffatomen steht und R wie in Anspruch 1 definiert ist, da-durch gekennzeichnet, daß man von einer Verbindung der Formel (II) ausgeht, worin R' die vorstehend für R'1 angegebenen Bedeutungen besitzt.

**3.** Verfahren gemäß Anspruch 1 zur Herstellung von Verbindungen der Formel (I), worin R für einen Phenyloder Naphthylrest steht, der durch einen oder mehrere Amino- oder Hydroxyreste substituiert ist, dadurch gekennzeichnet, daß man die entsprechenden Verbindungen der Formel (I), worin R eine oder mehrere Nitro-, Methoxy- oder Benzyloxygruppen bedeutet, reduziert.

**4.** Verfahren zur Herstellung der Verbindungen der Formel (I), wie in Anspruch 1 definiert, worin R die in Anspruch 1 angegebene Bedeutung besitzt und R' für ein Wasserstoffatom oder einen Acylrest mit 1 bis 6 Kohlenstoffatomen steht, dadurch gekennzeichnet, daß man ein 4-Pentenoylhalogenid der Formel (IV)

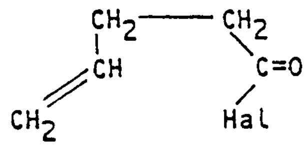

(IV)

worin Hal für ein Halogenatom steht, in Anwesenheit eines Kondensationsmittels der Einwirkung einer Verbindung der Formel (V)

$H_2N-SO_2-R$ (v)

unterzieht, um zu einer Verbindung der Formel (VI)

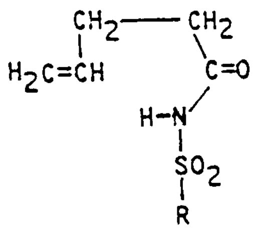

(VI)

zu gelangen, die man cyclisiert, um eine entsprechende Verbindung der Formel (I) zu erhalten, worin R' für ein Wasserstoffatom steht:

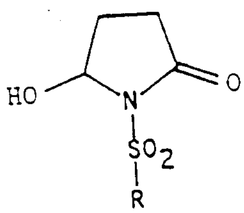

(I mit R' = H)

welche man gewünschtenfalls acyliert, um zu einer entsprechenden Verbindung der Formel (I) zu gelangen, worin R' für einen Acylrest steht:

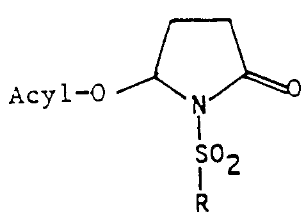

(I mit R' = Acyl).

**5.** Verfahren gemäß einem der Ansprüche 1 bis 4, dadurch gekennzeichnet, daß man von einer Verbindung der Formel (III) oder (V) ausgeht, worin R für eine gegebenenfalls durch einen Methoxyrest substituierte Phenylgruppe steht.

**6.** Verfahren gemäß einem der Ansprüche 1 bis 3, dadurch gekennzeichnet, daß man von einer Verbindung der Formel (II) ausgeht, worin R' für einen Alkylrest mit bis zu 8 Kohlenstoffatomen steht.

**7.** Verfahren gemäß Anspruch 6, dadurch gekennzeichnet, daß R' einen Ethylrest bedeutet.

**8.** Verfahren gemäß Anspruch 2, dadurch gekennzeichnet, daß man von einer Verbindung der Formel (II), worin $R^1$ für einen Ethylrest steht, und einer Verbindung der Formel (III), worin R für einen Phenylrest steht, ausgeht.

**9.** Verfahren gemäß Anspruch 2, dadurch gekennzeichnet, daß man von einer Verbindung der Formel (II), worin R' für einen Ethyl-, Isopropyl- oder Propylrest steht, und einer Verbindung der Formel (III), worin R für einen Phenyl- oder einen 4-Nitrophenylrest steht, ausgeht.

**10.** Verfahren zur Herstellung von pharmazeutischen Zusammensetzungen, dadurch gekennzeichnet, daß man als Wirkstoff zumindest eines der Produkte der Formel (I), wie in Anspruch 1 definiert, in eine für diese Verwendung bestimmte Form bringt.

**11.** Verfahren zur Herstellung von pharmazeutischen Zusammensetzungen, dadurch gekennzeichnet, daß man als Wirkstoff zumindest eines der Produkte der Formel (I'), wie in Anspruch 2 definiert, in eine für diese Verwendung bestimmte Form bringt.

**12.** Verfahren gemäß Anspruch 11, dadurch gekennzeichnet, daß in der Formel (I') R für einen Phenylrest steht, der gegebenenfalls durch einen der in Anspruch 1 definierten Substituenten substituiert ist, und $R'_1$ für einen Alkylrest mit bis zu 8 Kohlenstoffatomen steht.

**13.** Verfahren gemäß Anspruch 11, dadurch gekennzeichnet, daß der Wirkstoff das 1-(Benzolsulfonyl)-2-oxo-5-ethoxypyrrolidin ist.

**14.** Verfahren gemäß Anspruch 1, dadurch gekennzeichnet, daß das hergestellte Produkt das 5-Ethoxy-1-[-(2-thienyl)-sulfonyl]-2-pyrrolidinon oder das 5-Ethoxy-1-[(3-thienyl)-sulfonyl]-2-pyrrolidinon ist.